# EUROPEAN PATENT SPECIFICATION

(11) **EP 0 588 477 B1**
(45) Date of publication and mention of the grant of the patent: **15.10.1997**
(21) Application number: 93305602.0
(22) Date of filing: 16.07.1993
(51) Int. Cl.: A61K 38/18

(54) **Medicinal composition comprising TCF-II**
Arzneimittelzusammensetzung enthaltend TCF-II
Composition médicinale contenant du TCF-II

(30) Priority: 16.07.1992 JP 212227/92; 16.07.1992 JP 212229/92; 10.08.1992 JP 234198/92
(43) Date of publication of application: 23.03.1994
(60) Divisional application: 97100939.4
(73) Proprietor: SNOW BRAND MILK PRODUCTS CO., LTD., Sapporo-shi, Hokkaido 065 (JP)
(72) Inventor: Masunaga, Hiroaki, Shimotsuga-gun, Tochigi (JP); Fujise, Nobuaki, Ishibashimachi, Shimotsuga-gun, Tochigi (JP); Higashio, Kanji, Kawagoe-shi, Saitama (JP)
(74) Representative: Davies, Jonathan Mark

(56) References cited:
- EP-A- 0 461 560
- EP-A- 0 462 277
- EP-A- 0 539 590
- WO-A-91/03254
- US-A- 3 904 753
- DIALOG INFORMATION SERVICES, FILE 399: CA SEARCH, ACCESSION NUMBER 117:23902 & U.S. PAT.APPL. US 582063 (15.06.1991)
- PROC.NATL.ACAD.SCI.USA vol. 88 , January 1991 pages 415 - 419 J.S.RUBIN ET AL. 'A BROAD-SPECTRUM HUMAN...'
- PROC.NATL.ACAD.SCI.USA vol. 88 , August 1991 pages 7001 - 7005 K.M.WEIDNER ET AL. 'EVIDENCE FOR THE IDENTITY OF...'
- BIOCHEM.BIOPHYS.RES.COMMUN. vol. 172, no. 1 , 1990 pages 321 - 327 T.SEKI ET AL. 'ISOLATION AND EXPRESSION OF cDNA FOR DIFFERENT FORMS OF HEPATOCYTE GROWTH FACTOR FROM HUMAN LEUKOCYTE'

## Description

This invention relates to medicinal compositions containing on effective amount of TCF- II for the treatment of hypoproteinemia, involving the stimulation of protein synthesis.

Biologically active substances produced by human derived fibroblast cells, for example β -interferon as a tumor cytotoxic factor, have been well known.

Biologically active substances produced by fibroblast cells other than β -interferon such as a tumor cytotoxic glycoprotein called CBF in Japanese Unexamined Patent Publication No. 146293 (1983), a tumor cell proliferation inhibitor (INF) having a molecular weight of 35,000-45,000 in Japanese Unexamined Patent Publication No. 33120 (1986), a tumor proliferation factor (FNF) in Japanese Unexamined Patent Publication No. 1872 (1986), a physiologically active substance having a molecular weight of 40,000-60,000 and an isoelectric point of pH 5.0 ± 0.5 in Japanese Unexamined Patent Publication No. 103021 (1987), and a tumor cytotoxic factor having a molecular weight of 36,000 ± 1,000 and a specific amino acid sequence at isoelectric point of pH 10.5 or higher in Japanese Unexamined Patent Publication No. 10998 (1989), have been known. The inventors have been investigating antitumor substances derived from human fibroblast cells and found a new antitumor proteinous substance. Furthermore, the inventors successfully cloned a cDNA coding for the protein and determined its amino acid sequence. Also the usefulness of the protein was confirmed. The new antitumor protein and its gene were disclosed in the inventors' International Patent Publication No. 10651 (1990). The new antitumor protein was named TCF-II.

TCF-II has both potent antitumor activity and proliferation stimulative activity for normal cells and is a member of the HGF (hepatocyte growth factor) group. Molecular weight determination of TCF-II with SDS electrophoresis showed 78,000 ± 2,000 or 74,000 ± 2,000. The reduction products of TCF-II showed a common band (A chain) at 52,000 ± 2,000, and two bands (B and C chains) at 30,000 ± 2,000 and 26,000 ± 2,000, respectively.

TCF-II may be applied for the regeneration of liver after the hepatectomy due to its proliferative effect for hepatocytes.

Moreover, improvement or therapeutic effect in hypoproteinemia due to liver diseases, renal insufficiency or undernutrition has ever been found. Plasma protein is composed of more than 80 kinds of protein and most of their molecular weights range within 40,000-1,000,000. They often combine with carbohydrates or lipids to form conjugated proteins. These plasma proteins have important physiological significances such as blood coagulation factors, immunoglobulins, complements and enzymes, and also participate in the maintenance of osmotic pressure of plasma colloids and metabolism in the peripheral tissue. Most plasma proteins other than immunoglobulins are synthesized in the liver, released in the blood stream and widely distributed not only in the blood vessels but also in tissues and body cavity fluids, and actively exchange each other through lymph. Their catabolism includes secretion or excretion in gastrointestinal tract, kidneys, respiratory organs, reproductive organs and tear fluid, and degradation in the liver and reticuloendothelial system. The biological half life of plasma proteins is generally 2-20 days. The clinically serious hypoproteinemia occurs by various combinations of widely spread hemorrhage including ulcer and hematuria, decreased protein synthesis due to liver diseases, exhaustion and decreased production of plasma proteins accompanied by undernutrition due to all of nephrosis and nephritis, multiorgan failure, malignant tumors, infectious diseases, diabetes mellitus, gestosis and so forth.

Heretofore, intravenous administration of albumin preparations has been performed for the treatment of above mentioned hypoproteinemia providing a temporary improvement but neither complete remission nor effective treatment has been found.

### Summary of the Invention

The inventors noticed the biological activity of TCF-II and have been investigating the use of TCF-II as an antitumor agent and diagnostic marker of the diseases.

The inventors found that TCF-II provides not only proliferation of hepatocytes but also therapeutic effects on various liver diseases. Heretofore, the therapeutic effect of TCF-II on various liver diseases had not been confirmed.

The inventors found that TCF-II exhibits therapeutic effect on various hypoproteinemia and the therapeutic effect of TCF-II on hypoproteinemia is surprising.

The present invention provides the use of TCF-II in the preparation of a medicament for the treatment of hypoproteinemia.

The present invention results in a highly effective protein synthesis stimulator comprising an effective ingredient of TCF-II for the treatment of hypoproteinemia.

### Brief Description of the Drawings

Fig. 1 shows the increase of total plasma fibrinogen in hypoproteinemia rats after 70% liver resection followed by administration of the treatment agent provided by the present invention.

Fig. 2 shows the increase of total serum protein in hypoproteinemia rats after 70% liver resection followed by administration of the treatment agent provided by the present invention.

Fig. 3 shows the shortening of prothrombin time in hypoproteinemia rats after 70% liver resection followed by administration of the treatment agent provided by the present invention.

Fig. 4 shows the increase of plasma fibrinogen in hypoproteinemia rats due to DIC followed by administration of the treatment agent provided by the present invention.

Fig. 5 shows the increase of antithrombin III in hypoproteinemia rats caused by DIC followed by administration of the treatment agent provided by the present invention.

Fig. 6 shows the increase of total serum protein in hypoproteinemia rats caused by DIC followed by administration of the treatment agent provided by the present invention.

Fig. 7 shows the increase of total serum protein in hypoproteinemia rats caused by chronic renal failure followed by administration of the treatment agent provided by the present invention.

Fig. 8 shows the shortening of prothrombin time in hypoproteinemia rats caused by undernutrition followed by administration of the treatment agent provided by the present invention.

Fig. 9 shows the increase of antithrombin III activity in hypoproteinemia rats caused by undernutrition followed by administration of the treatment agent provided by the present invention.

In the Figs., * indicates P<0.05 and ** indicates P<0.01.

### Detailed Explanation of Preferred Embodiments

The effective ingredient of the present invention is a known glycoprotein (TCF-II) derived from human fibroblast cells as described previously.

TCF-II showed a molecular weight of 78,000 ± 2,000 or 74,000 ± 2,000 in the non-reduced state, and a common band A of 52,000 ± 2,000 and two bands B of 30,000 ± 2,000 and C of 26,000 ± 2,000 in the reduced state by SDS electrophoresis. TCF-II also showed an isoelectric point at pH 7.4-8.6 and was determined as a glycoprotein having a 723 amino acid sequence.

The above mentioned TCF-II can be obtained by evaporation of a human fibroblast cell culture solution, adsorption in an ion exchange resin and affinity chromatography of the eluate (WO90/10651) or by a genetic engineering, method (WO92/01053).

TCF-II can be obtained from human fibroblast cells cultured by the method disclosed in WO90/10651. Furthermore, TCF-II produced by a genetic recombination technique using microorganisms or other cells by the gene sequence disclosed in the above mentioned patent publication may be used. The production of TCF-II by the genetic engineering method may be carried out by the method invented by the present inventors and disclosed in WO92/01053. In addition, TCF-II analogues having different sugar chains or no sugar moieties produced by different host cells or microorganisms may also be used. However, presence of sugar moieties is preferable because of their participation in the in vivo metabolic rate.

TCF-II can be concentrated and purified by conventional isolation and purification methods, for example, precipitation with an organic solvent, salting out, gel filtration chromatography, affinity chromatography using a monoclonal antibody and electrophoresis. The purification by affinity chromatography using a monoclonal antibody disclosed in Japanese Patent Application No. 177236 (1991) by the present inventors may be applied.

The resultant purified TCF-II may be kept under lyophilization or deep freezing.

Furthermore, the protein synthesis stimulator resulting from the present invention may be administered as injection preparations and any route such as intravenous, intraarterial, intramuscular and subcutaneous injections can be selected. Blood coagulants such as fibrinogen, coagulation controlling factors such as antithrombin III and drugs such as FOY, a protease inhibitor gabexate mesylate, used for the treatment of DIC are also used concurrently.

The injection preparations of TCF-II may be used singly or in combination with above mentioned medicines and adjuvants such as human serum albumin, surface active agents, amino acids and sugars.

The doses of TCF-II included in the protein synthesis stimulants may be determined according to the symptoms, conditions and age of the patients but preparations containing 100-30,000 µg, preferably 500-3,000 µg of TCF-II are generally administered 1-7 times a week. Chronic administration may be appropriate according to the symptoms and conditions of the patients.

The present invention will be explained in more detail by the following examples.

### Example 1.

### Purification of TCF-II

Purified TCF-II was obtained by cell culture according to the method disclosed in WO90/10651 or Higashio, K. et al. (B.B.R.C., 170, 397-401, 1990).

Human fibroblast cells IMR-90 (ATCC CCL 186), 3 x 10⁶ cells, were inoculated in 100 ml of DMEM medium containing 5% bovine serum in a roller bottle and cultured at rotations of 0.5-2/min. for seven days. The culture was continued up to 1 x 10⁷ cells in total, the proliferated cells were separated by treatment with trypsin and collected at the bottom of the bottle. In the bottle, 100 g of sterilized 5-9 mesh ceramic (Toshiba Ceramic Co., Ltd.) was placed and cultured for 24 hrs. upon standing. Then, 500 ml of the culture medium shown above was added to the bottle and cultured further. The total culture medium was recovered every 7-10 days and fresh culture medium was supplied for further culture, Thus, the culture was continued for two months and four l/bottle of the culture solution was recovered.

The combined culture solution showed specific activity of 32 µ/ml.

Ultrafiltration of 750 l of the cultured solution was performed using a membrane filter (Amicon Corp., MW 6,000 cut) and the filtrate was chromatographed in five steps using CM Sephadex C-50 (Farmacia Biosystems Corp.), ConA Sepharose (Farmacia Biosystems Corp.), MonoS column (Farmacia Biosystems Corp.) and heparin Sepharose (Farmacia Biosystems Corp.) to give purified TCF-II having specific activity of 5,248,000 U/mg.

### Example 2

### Production of gene recombinant TCF-II

TCF-II gene recombinant cells were cultured according to the method disclosed in WO92/01053 and purified TCF-II was obtained. Transformed Namalwa cells were cultured and 20 l of the culture solution was obtained. The culture solution was treated successively with HPLC using CM-Sephadex C-50 chromato column, Con-A Sepharose CL-6B chromato column and MonoS column to give approximately 11 mg of active TCF-II.

### Example 3

### Production of pharmaceutical compositions of TCF-II

In the present examples, recombinant TCF-II obtained by Example 2 was used for the production of intravenous, subcutaneous and intramuscular injection preparations.

| | | |
|---|---|---|
| (1) | TCF-II | 40 µg |
| | Human serum Albumin | 1 mg |

The above composition was dissolved, in 0.01M PBS at pH 7.0 and adjusted to 20 ml in total. The solution was sterilized, divided into vials (2 ml each), lyophilized and sealed.

| | | |
|---|---|---|
| (2) | TCF-II | 40 µg |
| | Tween 80 | 1 mg |
| | Human serum albumin | 100 mg |

This composition was dissolved in a saline solution for injection and adjusted to 20 ml in total. The solution was sterilized, divided into vials (2 ml each), lyophilized and sealed.

| | | |
|---|---|---|
| (3) | TCF-II | 20 µg |
| | Tween 80 | 2 mg |
| | Sorbitol | 4 g |

This composition was dissolved in 0.01M PBS at pH 7.0 and adjusted to 20 ml in total. The solution was sterilized, divided into vials (2 ml each), lyophilized and sealed.

| | | |
|---|---|---|
| (4) | TCF-II | 40 µg |
| | Tween 80 | 2 mg |
| | Glycine | 2 g |

This composition was dissolved in a saline solution for injection and adjusted to 20 ml in total. The solution was sterilized, divided into vials (2 ml each), lyophilized and sealed.

| | | |
|---|---|---|
| (5) | TCF-II | 40 µg |
| | Tween 80 | 1 mg |
| | Sorbitol | 2 g |
| | Glycine | 1 g |

This composition was dissolved in a saline solution for injection and adjusted to 20 ml in total. The solution was sterilized, divided into vials. (2 ml each), lyophilized and sealed.

| | | |
|---|---|---|
| (6) | TCF-II | 20 µg |
| | Sorbitol | 4 g |
| | Human serum albumin | 50 mg |

This composition was dissolved in 0.01M PBS at pH 7.0 and adjusted to 20 ml in total. The solution was sterilized, divided into vials (2 ml each), lyophilized and sealed.

| | | |
|---|---|---|
| (7) | TCF-II | 40 µg |
| | Glycine | 2 g |
| | Human serum albumin | 50 mg |

This composition was dissolved in a saline solution for injection and adjusted to 20 ml in total. The solution was sterilized, divided into vials (2 ml each), lyophilized and sealed.

| | | |
|---|---|---|
| (8) | TCF-II | 10 mg |
| | Human serum albumin | 100 mg |

This composition was dissolved in 0.01M PBS at pH 7.0 and adjusted to 20 ml in total. The solution was sterilized, divided into vials (2 ml each), lyophilized and sealed.

These pharmaceutical preparations can be used as protein synthesis stimulants according to the above mentioned dosage and regimen. Hereinafter, test experiments with treatment agents prepared according to the present invention will be shown to confirm the therapeutic effects and explain the present invention.

The use of present invention results in protein synthesis stimulating agents comprising an effective ingredient of TCF-II for the treatment of hypoproteinemia. Hereinafter, experiments exhibiting the therapeutic effect of the agent prepared by the present examples will be shown to explain the effect of the present invention.

### Experiment 1

### Total serum protein increasing effect

### (1) Method

TCF-II was repeatedly and intravenously administered to seven week old male Wistar rats, six in one group, at doses of zero, five, 50, 500 and 5,000 µg/kg at 12 hr. interval for 14 days (28 times in total) and blood was drawn 12 hrs. after the final administration to determine total serum protein and serum albumin.

### (2) Results

TCF-II dose dependently increased the total serum protein (Fig. 14) and serum albumin (Fig. 15). Particularly, doses of five µg/kg or over significantly increased both parameters confirming the increasing effect on serum protein.

### Experiment 2

### Therapeutic effect of TCF-II on hypoproteinemia due to liver resection

Plasma protein lowered model rats were prepared by liver resection and the effect of TCF-II was confirmed using the model animals. In addition, plasma fibrinogen concentration and exogenous coagulation factor were noted as indicators of plasma protein and the changes in prothrombin time were noted as indicators of plasma protein including exogenous coagulation factors (Factors II, V, VII and X).

### (1) Method

Seven week old male Wistar rats, six in one group, were used for the experiment and 70% of the liver was resected. TCF-II was repeatedly and intravenously administered to the rats at doses of zero, 20, 100 and 500 µg/kg at 12 hr. intervals for two days, four times in total, and blood was drawn 48 hrs. after the start of the experiment to determine prothrombin time, plasma fibrinogen concentration and total serum protein.

### (2) Results

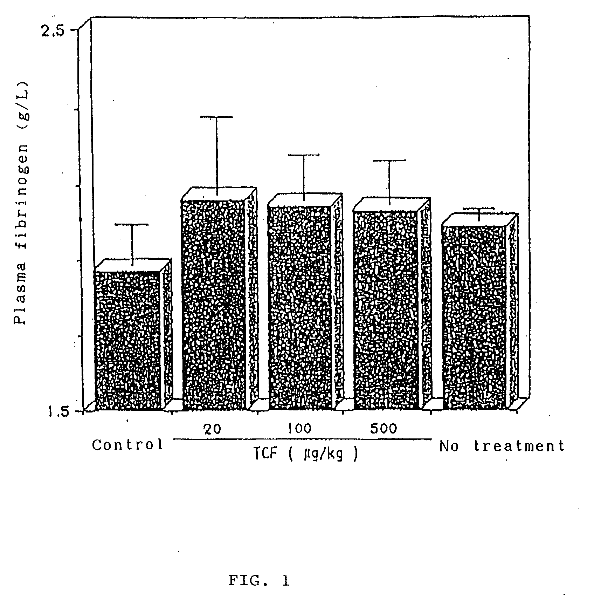

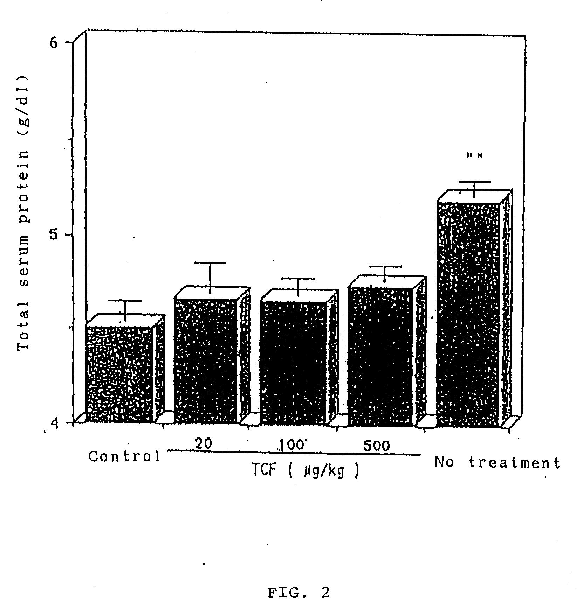

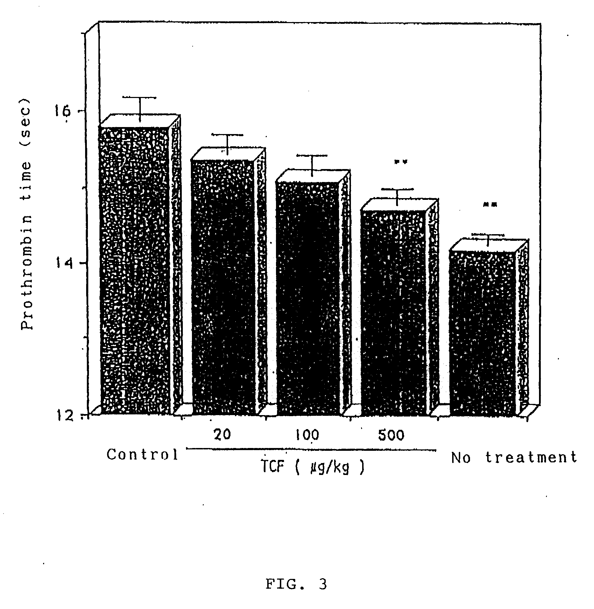

Shortening of prothrombin time (Fig. 3), increases of plasma fibrinogen concentration (Fig. 1) and total serum protein (Fig. 2) were observed with the administration of TCF-II. These results indicate the efficacy of the present invention for the treatment of hypoproteinemia due to liver damage.

### Experiment 3

### Therapeutic effect an hypoproteinemia caused by DIC

DIC causes sudden increase in consumption of plasma protein due to intravascular coagulation and reveals hypoproteinemia. The therapeutic agent of the present invention was given to model animals to confirm the therapeutic effect.

### (1) Method

Seven week old male Wistar rats, 10 in one group, were used for the experiment and 70% of the liver was resected. Immediately after the resection, 500 mg/kg of galactosamine was subcutaneously administered to prepare pathologic model of both liver damage and DIC symptoms. TCF-II was repeatedly and intravenously administered to the rats at a dose of 500 µg/kg at 12 hr. intervals for two days, four times in total, and blood was drawn 48 hrs. after the start of the experiment to determine plasma fibrinogen concentration, antithrombin III activity and total serum protein as indicators for the hypoproteinemia.

### (2) Results

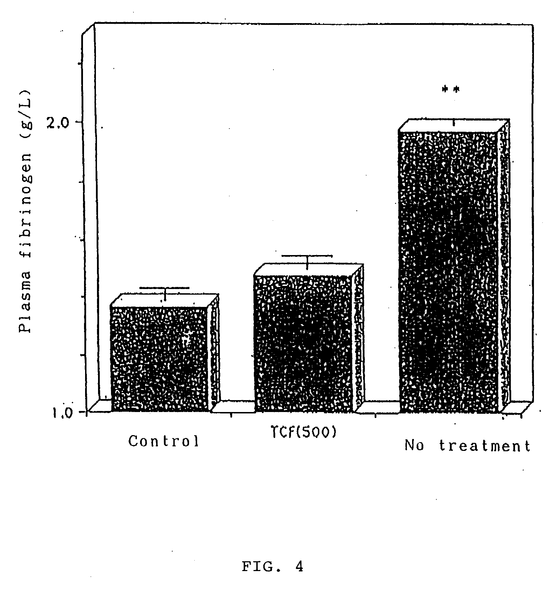

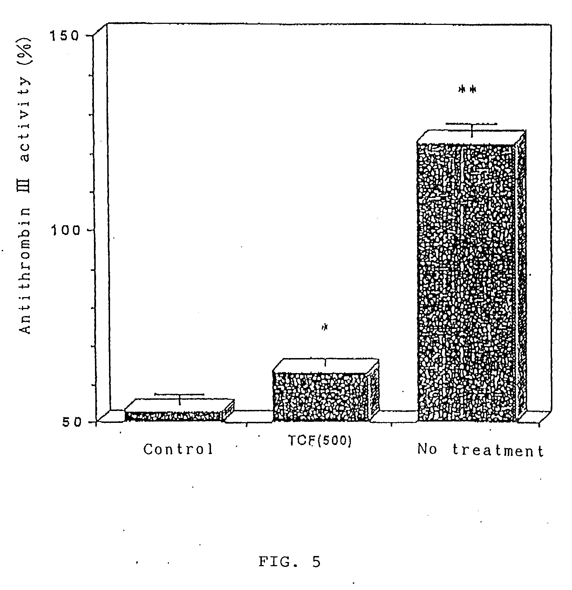

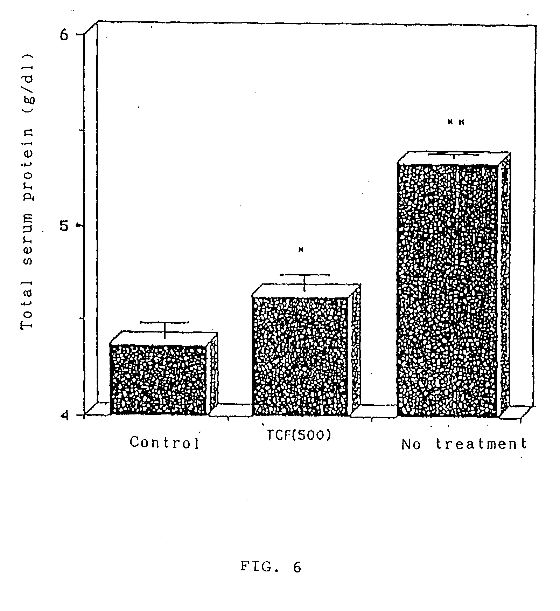

Increases of plasma fibrinogen (Fig. 4), antithrombin III activity (Fig. 5) and total serum protein (Fig. 6) were observed with the administration of TCF-II, confirming the efficacy of the present invention for the treatment of hypoproteinemia caused by DIC.

### Experiment 4

### Therapeutic effect on leaky hypoproteinemia caused by renal failure

### (1) Method

Eight week old male Wistar rats, 11 in one group, were used for the experiment and the kidneys were partially resected. After three weeks, TCF-II was repeatedly and intravenously administered to the rats at doses of zero and 500 µg/kg at 12 hr. intervals for 10 times in total, and blood was drawn 12 hrs. after the administration to determine total serum protein concentration.

### (2) Results

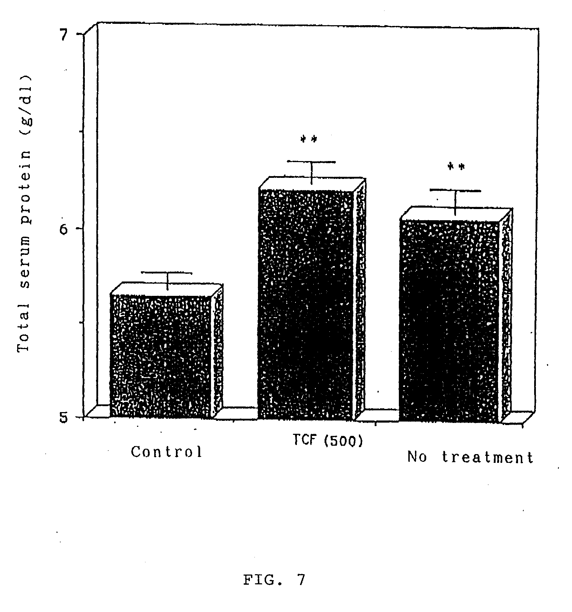

Increase of total serum protein was observed with the administration of TCF-II, confirming the efficacy of the present invention for the treatment of leaky hypoproteinemia caused by renal failure (Fig. 7).

### Experiment 5

### Therapeutic effect on undernutritional hypoproteinemia

### (1) Method

Essential amino acid deficient rats were prepared by replacing an essential amino acid methionine with a synthetic DL-ethionine.

Ethionine was repeatedly and intraperitoneally administered at a dose of 250 mg/kg/day for four days to prepare methionine deficient rat. The serum protein in the rats decreased due to amino acid deficiency. TCF-II was repeatedly and intravenously administered to the rats, 10 rats in one group, at doses of zero, 50, and 500 µg/kg every 12 hrs. for two days and four times in total; blood was drawn 48 hrs. after the start of the experiment and the prothrombin time and antithrombin III activity were determined as indicators of hypoproteinemia.

### (2) Results

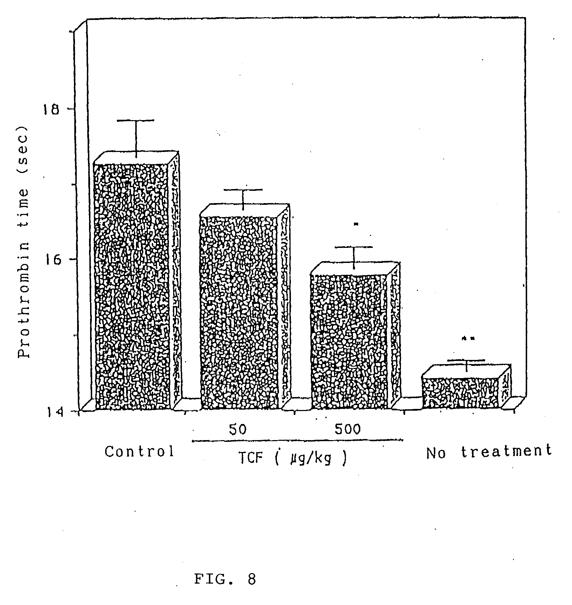

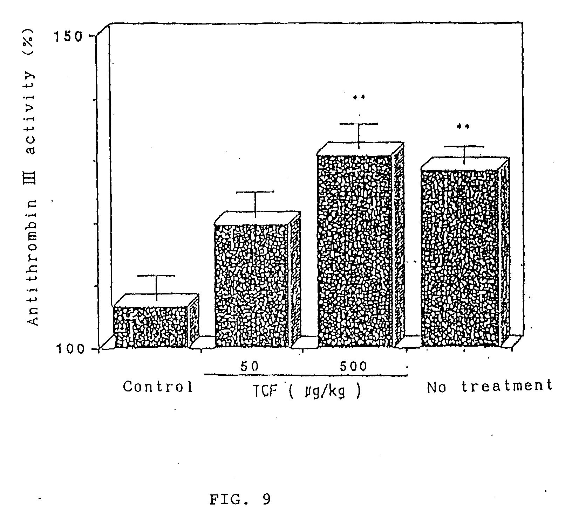

Shortening of prothrombin time (Fig. 8) and increase of antithrombin III activity (Fig. 9) were observed with the administration of TCF-II, confirming the efficacy of the present invention for the treatment of hypoproteinemia due to undernutrition.

These results confirm the efficacy of the treatment agent produced by the present invention on hypoproteinemia.

## Claims

1. Use of TCF-II in the preparation of a medicament for the treatment of hypoproteinemia.

2. Use according to claim 1 in the preparation of a medicament for the treatment of hepatopathic hypoproteinemia, leaky hyproproteinemia caused by renal failure or undernutritional hyproproteinemia.

3. Use according to claim 1 or 2 in which the medicament prepared is in injectable form.

## Patentansprüche

1. Verwendung von TCF-II bei der Herstellung eines Medikaments zur Behandlung von Hypoproteinämie.

2. Verwendung nach Anspruch 1 bei der Herstellung eines Medikaments zur Behandlung von hepatopathischer Hypoproteinämie, durch Nierenversagen verursachter mit Inkontinenz verbundener (leaky) Hypoproteinämie oder durch Unterernährung verursachter Hypoproteinämie.

3. Verwendung nach Anspruch 1 oder 2, wobei das hergestellte Medikament in injizierbarer Form vorliegt.

## Revendications

1. Utilisation de TCF-II dans la préparation d'un médicament pour le traitement de l'hypoprotéinémie.

2. Utilisation selon la revendication 1 dans la préparation d'un médicament pour le traitement de l'hypoprotéinémie hépatopathique, de l'hypoprotéinèmie avec fuite provoquée par une déficience rénale ou de l'hypoprotéinémie par sous-nutrition.

3. Utilisation selon la revendication 1 ou 2, dans laquelle le médicament préparé est sous forme injectable.
